# EUROPÄISCHE PATENTANMELDUNG

(11) **EP 1 121 900 A2**
(43) Veröffentlichungstag der Anmeldung: **08.08.2001**
(21) Anmeldenummer: 01200315.8
(22) Anmeldetag: 29.01.2001
(51) Int. Cl.: A61B 6/12

(54) **Verfahren zur Positionsbestimmung eines medizinischen Instruments**

(30) Priorität: 03.02.2000 DE 10004764
(71) Anmelder: Philips Corporate Intellectual Property GmbH, 52064 Aachen (DE); Koninklijke Philips Electronics N.V., 5621 BA Eindhoven (NL)
(72) Erfinder: Zylka, Waldemar, Dr., Habsburgerallee 11, 52064 Aachen (DE); Sabczynski, Jorg, Dr., Habsburgerallee 11, 52064 Aachen (DE); Weese, Jürgen, Dr., Habsburgerallee 11, 52064 Aachen (DE)
(74) Vertreter: Gössmann, Klemens

(57) **Zusammenfassung**

Die Erfindung betrifft ein Verfahren und eine Anordnung zur Bestimmung der Position eines in ein Untersuchungsobjekt teilweise eingerührten medizinischen Instruments (13) in einem dreidimensionalen Bilddatensatz (CT) des Untersuchungsobjekts. Um einerseits eine möglichst hohe Genauigkeit der Positionsbestimmung zu erreichen und andererseits den erforderlichen Aufwand möglichst gering zu halten, insbesondere aufwendige Registrierungsschritte vor einem Eingriff einzusparen, wird erfindungsgemäß vorgeschlagen, gleichzeitig zur Erfassung von Röntgenbildern (I_{r,}) deren räumliche Positionen und die räumliche Position eines verwendeten medizinischen Instruments (6) zu erfassen, anschließend eine räumliche Zuordnung zwischen einem Röntgenbild (Iᵣ) und dem dreidimensionalen Bilddatensatz (CT) zu bestimmen und diese auf geeignete Weise dazu auszunutzen, die räumliche Position des medizinischen Instruments (16) in eine Position relativ zu dem dreidimensionalen Bilddatensatz (CT) umzurechnen. Dadurch können Bilder erstellt werden, die sowohl präoperativ als auch intraoperativ erfasste Bildinformationen enthalten und in die die aktuelle Position des medizinischen Instruments eingeblendet ist

## Beschreibung

Die Erfindung betrifft ein Verfahren zur Bestimmung der Position eines in ein Untersuchungsobjekt teilweise eingeführten medizinischen Instruments in einem dreidimensionalen Bilddatensatz des Untersuchungsobjekts sowie eine Anordnung zur Durchführung dieses Verfahrens.

Ein Verfahren und eine Anordnung zur Bestimmung der Position eines medizinischen Instruments in einem zweidimensionalen Bilddatensatz sind aus der EP 857 461 A2 bekannt. Dabei werden mittels einer C-Bogen-Röntgeneinrichtung intraoperativ Röntgenbilder des Untersuchungsbereichs eines Untersuchungsobjekts, z.B. eines Patienten, erfasst, wobei gleichzeitig mittels einer optischen Positionsmesseinrichtung die Position des Untersuchungsobjekts bzw. des Patiententisches und des medizinischen Instruments relativ zu der Röntgeneinrichtung gemessen wird. Die Position des medizinischen Instruments kann anschließend in eine Position relativ zu einem oder mehreren der erfassten Röntgenbildern umgerechnet werden, so dass die aktuelle Position jeweils in einem oder mehreren Röntgenbildern angezeigt werden kann. Ein derartiges Verfahren kann als Navigationshilfe für den Arzt während der Behandlung eines Patienten dienen. Dabei besteht jedoch der Nachteil, dass intraoperativ für die Navigation keine dreidimensionalen Bildinformationen zur Verfügung stehen. Zwar ist es möglich, eine Intervention anhand eines präoperativen dreidimensionalen Datensatzes zu planen, intraoperativ können jedoch immer nur zweidimensionale Röntgenbilder erfasst werden, und die Position des medizinischen Instruments kann immer nur in diesen intraoperativ erfassten zweidimensionalen Röntgenbildern bestimmt und angezeigt werden.

Zwar sind auch Verfahren bekannt, bei denen die Position eines medizinischen Instruments intraoperativ bestimmt wird und in eine Position relativ zu einem präoperativ erfassten dreidimensionalen Bilddatensatz umgerechnet werden kann. Dazu müssen jedoch bei der Erfassung des dreidimensionalen Bilddatensatzes präoperativ spezielle Marker an dem Patienten angebracht sein, die in dem dreidimensionalen Bilddatensatz mit abgebildet werden und die unmittelbar vor der Operation mittels eines speziellen Pointers angefahren werden, um deren räumliche Positionen zu bestimmen. Mittels der dann in räumlichen Koordinaten und in 3D-Bildkoordinaten bekannten Positionen dieser Marker kann dann eine intraoperativ gemessene räumliche Position eines medizinischen Instruments in eine Position relativ zu dem dreidimensionalen Bilddatensatz umgerechnet werden. Derartige Verfahren haben jedoch den Nachteil, dass keine aktuellen Bildinformationen der Anatomie des Patienten verwendet werden und dass die Anatomie während der Intervention regelmäßig verändert ist gegenüber dem Zustand der Anatomie bei der präoperativen Erfassung des 3D-Bilddatensatzes.

Der Erfindung liegt deshalb die Aufgabe zugrunde, ein Verfahren zur Bestimmung der Position eines in ein Untersuchungsobjekt teilweise eingeführten medizinischen Instruments in einem dreidimensionalen Bilddatensatz des Untersuchungsobjekts anzugeben, bei dem die genannten Nachteile vermieden werden, wobei insbesondere eine hohe Genauigkeit bei möglichst geringem Aufwand erreicht werden soll. Außerdem soll eine entsprechend ausgestaltete Anordnung zur Durchführung des Verfahrens angegeben werden.

Diese Aufgaben werden durch ein Verfahren gemäß Anspruch 1 bzw. durch eine Anordnung gemäß Anspruch 9 gelöst.

Die Erfindung geht dabei von der Erkenntnis aus, dass ein intraoperatives zweidimensionales Röntgenbild vorteilhaft dazu ausgenutzt werden kann, die ebenfalls intraoperativ gemessene Position des medizinischen Instruments in eine Position relativ zu einem dreidimensionalen Bilddatensatz, der in der Regel präoperativ erfasst sein wird, umzurechnen. Erfindungsgemäß wird dazu intraoperativ neben der Position des medizinischen Instruments auch die räumliche Position des Röntgenbildes bestimmt. Mittels eines geeigneten Registrierungsverfahrens wird anschließend die räumliche Zuordnung zwischen diesem Röntgenbild und dem dreidimensionalen Bilddatensatz bestimmt, woraus sich quasi die räumliche Position des dreidimensionalen Bilddatensatzes ergibt. Mit Hilfe dieser Kenntnis ist es nun einfach möglich, die Position des medizinischen Instruments relativ zu dem dreidimensionalen Bilddatensatz zu bestimmen, da ja die räumliche Position des medizinischen Instruments unmittelbar vorher erfasst worden ist. Mit der Erfindung ist es somit auf einfacher Weise möglich, die Position eines medizinischen Instruments in einem dreidimensionalen Bilddatensatz zu bestimmen, ohne dass bei der Erfassung des Bilddatensatzes spezielle mit abzubildende Marker an dem Patienten angebracht werden müssen, die unmittelbar vor der Operation nochmals registriert werden müssen. Außerdem werden dabei intraoperativ erfasste Bilddaten verarbeitet, die genaue Aussagen über die Anatomie des Patienten zulassen, wodurch die Genauigkeit der Positionsbestimmung erhöht wird.

Die vorteilhafte Ausgestaltung gemäß Anspruch 2 nutzt bekannte Mittel zur Bestimmung der räumlichen Position des Röntgenbildes, die auch zur Bestimmung der räumlichen Position des medizinischen Instruments verwendet werden können. Die dazu verwendete Positionsmesseinrichtung kann unterschiedlich ausgestaltet sein, beispielsweise mit optischen Kameras, Infrarot-Kameras und/oder elektromagnetischen Detektoren, die die dreidimensionale Position von korrespondierenden Markern, beispielsweise optischen Leuchtdioden, Infrarot-Dioden oder elektromagnetischen Sendern, bestimmen können.

Die Weiterbildungen gemäß den Ansprüchen 3 bis 5 stellen Möglichkeiten dar zur Bestimmung der räumlichen Zuordnung zwischen dem Röntgenbild und dem dreidimensionalen Bilddatensatz. Dazu wird der dreidimensionale Bilddatensatz insgesamt, ein oder mehrere Teilvolumina, einzelne in dem Bilddatensatz enthaltene besonders auffällige Objekte oder Strukturen oder auch einzelne Voxel des Bilddatensatzes mit dem Röntgenbild verglichen bzw. in dem Röntgenbild gesucht. Dies erfolgt bevorzugt iterativ. Eine solche vorteilhafte Vergleichsmethode ist in Anspruch 5 angegeben und aus der EP 880 109 A2 bekannt, auf die hiermit ausdrücklich verwiesen sei und deren Offenbarungsgehalt als in die vorliegende Anmeldung mit aufgenommen gelten soll. Dabei werden aus dem dreidimensionalen Bilddatensatz Pseudo-Projektionsbilder erstellt und mit dem Röntgenbild verglichen, wobei die der Erstellung des Pseudo-Projektionsbildes zugrunde liegenden Parameter wie Abbildungsmaßstab, Projektionsrichtung usw. so lange iterativ variiert werden, bis das Pseudo-Projektionsbild und das Röntgenbild möglichst optimal übereinstimmen. Dann ist die räumliche Zuordnung zwischen Röntgenbild und 3D-Bilddatensatz gefunden.

Bevorzugt ist gemäß Anspruch 6 das Verfahren intraoperativ und kontinuierlich durchführbar ausgestaltet, so dass es während der Behandlung eines Patienten dem Arzt als Navigationshilfe dienen kann und ständig aktuelle Informationen über die Anatomie und die Position des medizinischen Instruments liefern kann.

Gemäß der vorteilhaften Weiterbildung nach Anspruch 7 wird aus dem dreidimensionalen Bilddatensatz ein Bild erstellt, in das die Position des medizinischen Instruments oder das Instrument selbst eingeblendet ist. Dies dient ebenfalls der Unterstützung des behandelnden Arztes während einer Operation. Es können dabei unterschiedliche Bilder dargestellt werden, z.B. Schichtbilder oder Projektionsbilder, die aus dem dreidimensionalen Bilddatensatz erstellt wurden und sich mittels der intraoperativ benutzten Röntgeneinrichtung nicht erstellen lassen, wie z.B. Kombinationsbilder aus präoperativ erfassten und intraoperativ erfassten Bilddaten, Gefäßbäume oder präoperativ erstellte Navigationspläne.

Besonders vorteilhaft ist die Ausgestaltung gemäß Anspruch 8, bei der der dreidimensionale Bilddatensatz präoperation mittels einer beliebigen bildgebenden Einrichtung erfasst wurde und bei der intraoperativ eine Röntgenfluroskopieeinrichtung, z.B. eine C-Bogen-Röntgeneinrichtung, benutzt wird. Dadurch können dem Arzt während der Behandlung auch Bilder aus verschiedenen Bildgebungsmodalitäten mit unterschiedlichem Informationsgehalt intraoperativ präsentiert werden.

Eine erfindungsgemäße Anordnung, mit der sich das beschriebene Verfahren besonders vorteilhaft durchführen lässt, mit einer Röntgeneinrichtung, einer Positionsmesseinrichtung und einer Recheneinheit ist in Anspruch 9 angegeben.

Die Erfindung wird nachfolgend anhand der Zeichnungen näher erläutert. Es zeigen:
Fig. 1 eine schematische Darstellung einer erfindungsgemäßen Anordnung,
Fig. 2 ein Diagramm zur Erläuterung des Verfahrensablaufs und
Fig. 3 ein Blockschaltbild zur Erläuterung des erfindungsgemäßen Verfahrens.

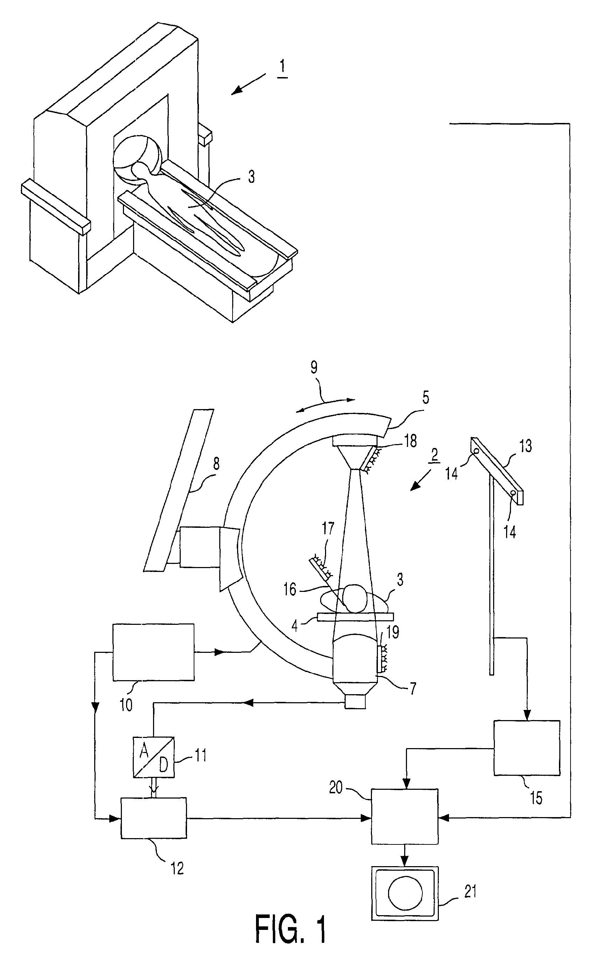

Fig. 1 zeigt einen Computertomographen 1, mit dem von einem Patienten 3 vor Beginn eines chirurgischen Eingriffs eine Folge von Computertomogrammen angefertigt wird, die parallele zur Längsachse des Patienten 3 senkrechte Schichten darstellen. Diese Computertomogramme bilden einen dreidimensionalen Bilddatensatz zur dreidimensionalen Darstellung des Untersuchungsbereichs des Patienten 3. Anhand dieses Bilddatensatzes lässt sich beispielsweise der später auszuführende chirurgische Eingriff genau planen.

Mittels einer Röntgeneinrichtung 2 werden während des Eingriffs laufend zweidimensionale Röntgenbilder des auf einem Operationstisch 4 angeordneten Patienten 3 erstellt. Dazu wird vorliegend eine C-Bogen-Röntgeneinrichtung verwendet, bei der eine Röntgenquelle 6 und ein Röntgendetektor 7 an einem C-Bogen 5 angeordnet sind, der von einem nicht näher dargestellten Stativ 8 getragen wird. Der C-Bogen ist dabei wenigstens in Pfeilrichtung 9 um eine horizontale Achse verschwenkbar. Der Röntgenbildaufnehmer 7 liefert seine Ausgangssignale über den Analog-Digital-Wandler 11 zu einem Bildspeicher 12, der mit einer Recheneinheit 20 verbunden ist. Die Steuerung der Röntgeneinrichtung 2 erfolgt mittels einer Steuereinheit 10.

Weiter ist eine Positionsmesseinrichtung 13 mit zwei Infrarot-CCD-Kameras 14 vorgesehen, die seitlich neben dem Untersuchungsbereich auf einem Stativ angeordnet sind. Mit dieser lassen sich räumliche Positionen von entsprechend ausgestalteten Infrarot-Leuchtdioden bestimmen. Um die Position eines während des Eingriffs verwendeten medizinischen Instruments 16, hier einer Biopsienadel, zu bestimmen, sind an dem aus dem Patienten herausragenden Ende der Biopsienadel 16 an definierten Positionen drei solche Infrarot-Leuchtdioden 17 angeordnet. Um weiter die Position der Röntgeneinrichtung 2 bzw. die Abbildungsgeometrie der Röntgeneinrichtung 2 bei der Erstellung von Röntgenbildern während der Operation zu bestimmen, sind jeweils an der Röntgenquelle 6 und an dem Röntgen detektor 7 drei solche Leuchtdioden 18 bzw. 19 angeordnet. Aus der so bestimmten Abbildungsgeometrie lässt sich die räumliche Position eines erfassten Röntgenbildes bestimmen, d.h. die Lage des Röntgenbildes relativ zu dem Patienten 3. Diese Berechnung und die Speicherung der ermittelten Positionen erfolgt in einer Positionsrecheneinheit 15, dessen Ergebnisse wiederum an die Recheneinheit 20 weitergegeben werden.

Der Recheneinheit 20 wird neben den intraoperativ erfassten Röntgenbildern und den gemessenen Positionen auch der von dem Computertomographen 1 präoperativ erfasste Bilddatensatz zugeführt. Dort wird aus diesen Daten mittels einer noch näher zu erläuternden Vergleichsmethode die räumliche Zuordnung zwischen dem zweidimensionalen Röntgenbild und dem dreidimensionalen Bilddatensatz bestimmt. Nach Bestimmung dieser Zuordnungsvorschrift ist es möglich, die räumliche Position des medizinischen Instruments 16 in eine Position relativ zu dem dreidimensionalen Bilddatensatz umzurechnen und aus dem dreidimensionalen Bilddatensatz und/oder dem intraoperativ erstellten Röntgenbild ein oder mehrerer Bilder zu erstellen, die auf einem Monitor 21 dargestellt werden können und in die die Position des medizinischen Instruments eingeblendet sein kann.

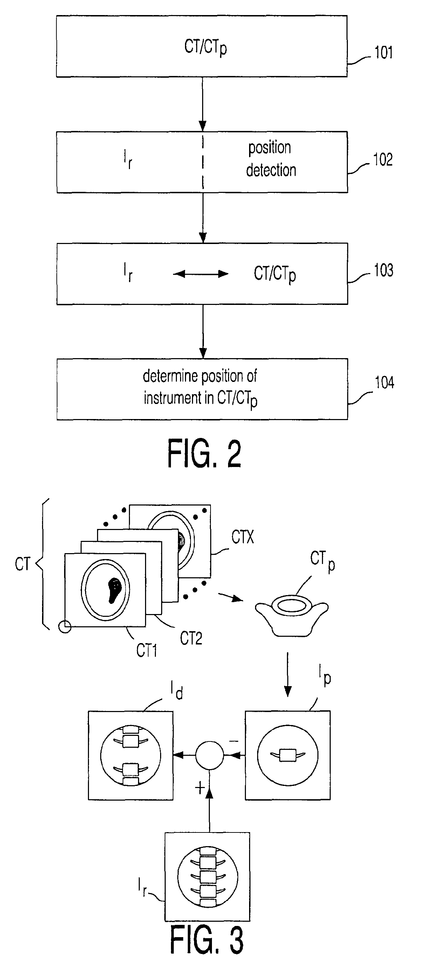

In Fig. 2 sind die einzelnen Verfahrensschritte noch einmal in einem Ablaufdiagramm dargestellt. In dem präoperativ durchgeführten Verfahrensschritt 101 wird ein dreidimensionaler Bilddatensatz CT mit Hilfe des Computertomographen erfasst, der die Absorptionsverteilung innerhalb eines Untersuchungsvolumens dreidimensional wiedergibt. Aus diesem kann auch ein Teilvolumen CTₚ ausgewählt werden, das einen für den späteren Eingriff besonders interessierenden Bereich umfasst. Diese Auswahl kann manuell oder automatisch durch Segmentierung erfolgen.

Die nachfolgend gezeigten intraoperativ durchgeführten Verfahrensschritte 102 bis 104 können während eines Eingriffs kontinuierlich oder mehrfach in gewünschten Zeitabständen oder zu bestimmten Zeitpunkten durchgeführt werden. Im Verfahrensschritt 102 werden gleichzeitig ein zweidimensionales Röntgenbild Iᵣ und die Positionen der Abbildungsgeometrie und des medizinischen Instruments bestimmt. Zwischen dem Röntgenbild Iᵣ und dem Gesamtvolumen CT bzw. dem Teilvolumen CTₚ des dreidimensionalen Bilddatensatzes wird anschließend im Verfahrensschritt 103 die Zuordnungsvorschrift bestimmt. Diese wird schließlich im Verfahrensschritt 104 dazu benutzt, um die Position des medizinischen Instruments in dem Gesamtvolumen CT bzw. dem Teilvolumen CTₚ des Bilddatensatzes zu bestimmen und ggf. geeignete Bilder zu erstellen.

Anhand von Fig. 3 soll die Vergleichsmethode zur Bestimmung der Zuordnungsvorschrift näher erläutert werden. Der dreidimensionale Bilddatensatz CT wird dabei aus mehreren von dem Computertomographen erfassten Schichtbildern CT1, CT2, ..., CTX gebildet.

Daraus wird ein Teilvolumen CTₚ ausgewählt, das für den späteren Eingriff relevant ist und im gezeigten Fall beispielsweise einen Wirbelkörper darstellt.

Die räumliche Transformation bzw. Zuordnung zwischen der Abbildung des Patienten, insbesondere des segmentierten Wirbelkörpers, durch den CT-Datensatz und der Lage dieses Wirbelkörpers im Raum wird mit Hilfe der intraoperativ erfassten Röntgenaufnahme Iᵣ ermittelt. Dazu werden von dem Teilvolumen CTₚ Pseudo-Projektionsbilder Iₚ erzeugt. Die Größe des Pseudo-Projektionsbildes Iₚ soll dabei der Größe des Röntgenbildes Iᵣ entsprechen. Die Lage des Projektionspunktes, von dem aus das Teilvolumen CTₚ in das Pseudo-Projektionsbild Iₚ projiziert wird, entspricht der Lage der Röntgenquelle (bzw. des die Röntgenstrahlung emittierenden Brennflecks der Röntgenquelle) in bezug auf den Röntgendetektor bei der Röntgenaufnahme. In der Regel stimmen die zunächst gewählte Ausgangsposition des Teilvolumens CTₚ in bezug auf den Projektionspunkt und die Projektionsrichtung nicht mit der Position und der Ausrichtung des realen Teilvolumens bei der Erzeugung der Röntgenaufnahme in bezug auf die Röntgenquelle und den Röntgendetektor überein. Deshalb werden diese Projektionsparameter des Teilvolumens CTₚ in bezug auf den Projektionspunkt und die Ebene des Projektionsbildes Iₚ so lange verändert, bis sich in einem aus der Differenz zwischen dem Röntgenbild Iᵣ und dem Pseudo-Projektionsbild Iₚ abgeleiteten Differenzbild I_{d} die Abbildung des Wirbelkörpers CTₚ möglichst gut auslöscht. Dies ist der Fall, wenn die dem Pseudo-Projektionsbild Iₚ zugrunde gelegte Lage und Ausrichtung des Wirbelkörpers CTₚ mit der Lage und der Ausrichtung des realen Wirbelkörpers in bezug auf die Röntgenquelle und den Röntgendetektor übereinstimmt. Hinsichtlich einer weiteren Erläuterung dieses Verfahrens sei nochmals auf die EP 880 109 A2 verwiesen.

Mit dem beschriebenen Verfahren lässt sich die Zuordnung zwischen dem Röntgenbild Iᵣ und dem dreidimensionalen Bilddatensatz CT in den zwei zur Röntgenstrahlungsrichtung senkrechten Richtungen sehr genau bestimmen. In Richtung des zentralen Röntgenstrahls ist die Bestimmung jedoch wesentlich ungenauer. Dies lässt sich jedoch dadurch verbessern, dass eine zweite Röntgenaufnahme mit einem zur ersten Röntgenaufnahme senkrechten Strahlengang angefertigt wird und das Vergleichsverfahren auch mit dieser zweiten Röntgenaufnahme ausgeführt wird.

Die Erfindung ist nicht auf die gezeigte Ausführungsform beschränkt, die lediglich beispielhaft zu verstehen ist. Der dreidimensionale Bilddatensatz kann auch mittels eines anderen bildgebenden Systems erfasst sein, des weiteren können die intraoperativ benutzte Röntgeneinrichtung und das Positionsmesseinrichtung anders ausgestaltet sein, sofern die geforderte Funktionalität erfüllt ist. Auch die konkrete Ausgestaltung der Verfahrensschritte, insbesondere die Bestimmung der Zuordnungsvorschrift zwischen dem dreidimensionalen Bilddatensatz und den zweidimensionalen Röntgenbildern, kann völlig anders erfolgen. Die beschriebene Vergleichsmethode ist lediglich ein Beispiel für die Bestimmung dieser Zuordnung.

## Patentansprüche

1. Verfahren zur Bestimmung der Position eines in ein Untersuchungsobjekt (3) teilweise eingeführten medizinischen Instruments (13) in einem dreidimensionalen Bilddatensatz (CT) des Untersuchungsobjekts (3) mit den Verfahrensschritten:
• Erfassung eines zweidimensionalen Röntgenbildes (Iᵣ) des Untersuchungsobjekts (3) mittels einer Röntgeneinrichtung (2),
• Erfassung der räumlichen Positionen des Röntgenbildes (Iᵣ) und des medizinischen Instruments (16),
• Bestimmung der räumlichen Zuordnung zwischen dem Röntgenbild (Iᵣ) und dem dreidimensionalen Bilddatensatz (CT) und
• Bestimmung der Position des medizinischen Instruments (16) in dem dreidimensionalen Bilddatensatz (CT) aus der räumlicher Position des medizinischen Instruments (16) mittels der räumlichen Zuordnung zwischen dem Röntgenbild (Iᵣ) und dem dreidimensionalen Bilddatensatz (CT).

2. Verfahren nach Anspruch 1,
dadurch gekennzeichnet,
dass die räumliche Position des Röntgenbildes (Iᵣ) dadurch erfasst wird, dass die räumlichen Positionen der bildgebenden Elemente (6, 7) der Röntgeneinrichtung (2), insbesondere der Röntgenquelle (6) und des Röntgendetektors (7), mittels einer Positionsmesseinrichtung (13) erfasst werden.

3. Verfahren nach Anspruch 1,
dadurch gekennzeichnet,
dass die räumliche Zuordnung zwischen dem Röntgenbild (Iᵣ) und dem dreidimensionalen Bilddatensatz (CT) mittels einer Vergleichsmethode bestimmt wird.

4. Verfahren nach Anspruch 3,
dadurch gekennzeichnet,
dass bei der Vergleichsmethode ein Teilvolumen (CTₚ) des dreidimensionalen Bilddatensatzes (CT) mit dem Röntgenbild verglichen und eine Zuordnungsvorschrift iterativ bestimmt wird.

5. Verfahren nach Anspruch 3 oder 4,
dadurch gekennzeichnet,
dass bei der Vergleichsmethode aus wenigstens einem Teildatensatz (CTₚ) des dreidimensionalen Bilddatensatzes (CT) ein Pseudo-Projektionsbild (Iₚ) bestimmt wird, dass das Pseudo-Projektionsbild (Iₚ) mit dem Röntgenbild (Iᵣ) verglichen wird und dass die der Bestimmung des Pseudo-Projektionsbildes (Iₚ) zugrunde liegenden Parameter iterativ solange verändert werden, bis sich eine optimale Übereinstimmung zwischen dem Pseudo-Projektionsbild (Iₚ) und dem Röntgenbild (Iᵣ) ergibt.

6. Verfahren nach Anspruch 1,
dadurch gekennzeichnet,
dass das Verfahren intraoperativ und kontinuierlich durchgeführt wird.

7. Verfahren nach Anspruch 1,
dadurch gekennzeichnet,
dass ein aus dem dreidimensionalen Bilddatensatz (CT) erstelltes Bild dargestellt wird, in das die Position des medizinischen Instruments (16) oder das Instrument (16) selbst eingeblendet ist.

8. Verfahren nach Anspruch 1,
dadurch gekennzeichnet,
dass das Röntgenbild (Iᵣ) mittels einer Röntgenfluoroskopieeinrichtung (2) erfasst wird und dass der dreidimensionale Bilddatensatz (CT) mittels eines Computertomographen (1),
einer Kernspintomographieeinrichtung, einer Ultraschalleinrichtung oder einer Röntgeneinrichtung präoperativ erfasst wurde.

9. Anordnung zur Bestimmung der Position eines in ein Untersuchungsobjekt (3) eingeführten medizinischen Instruments (16) in einem dreidimensionalen Bilddatensatz (CT) des Untersuchungsobjekts (3) mit:
• einer Röntgeneinrichtung (2) zur Erfassung eines zweidimensionalen Röntgenbildes (Iᵣ) des Untersuchungsobjekts (3),
• einer Positionsmesseinrichtung (13) zur Erfassung der räumlichen Positionen des Röntgenbildes (Iᵣ) und des medizinischen Instruments (16) und
• einer Recheneinheit (20) zur Bestimmung der räumlichen Zuordnung zwischen dem Röntgenbild (Iᵣ) und dem dreidimensionalen Bilddatensatz (CT) und zur Bestimmung der Position des medizinischen Instruments (16) in dem dreidimensionalen Bilddatensatz (CT) aus der räumlicher Position des medizinischen Instruments (16) mittels der räumlichen Zuordnung zwischen dem Röntgenbild (Iᵣ) und dem dreidimensionalen Bilddatensatz (CT).
